(19) Europäisches Patentamt
European Patent Office
Office européen des brevets

(11) **EP 4 060 676 A1**

(12) **EUROPEAN PATENT APPLICATION**

(43) Date of publication:
**21.09.2022 Bulletin 2022/38**

(21) Application number: **21162964.7**

(22) Date of filing: **16.03.2021**

(51) International Patent Classification (IPC):
***G16H 50/00*** *(2018.01)*

(52) Cooperative Patent Classification (CPC):
**G16H 50/70; G06N 3/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **PPRS SAS**
**68000 Colmar (FR)**

(72) Inventors:
• **Muller, Bruno**
  **57410 Rohrbach-lès-Bitche (FR)**
• **Lengellé, Régis**
  **10440 Torvilliers (FR)**

(74) Representative: **Bird & Bird LLP**
**Am Sandtorkai 50**
**20457 Hamburg (DE)**

(54) **A COMPUTER-IMPLEMENTED METHOD OF SELECTING A PREFERRED TRAINING DATASET FROM A PLURALITY OF TRAINING DATASETS FOR A TARGET DATASET**

(57) Described and claimed is a computer-implemented method of selecting a preferred training dataset from a plurality of training datasets for a target dataset by applying for each training dataset a reference detector to the target dataset to obtain a reference output, determining for each training dataset a performance indicator for the reference detector based on the reference output, the data points of the target dataset and the kernel or similarity function of the respective training dataset and selecting the preferred training dataset based on the performance indicator. Further, a data processing system implementing the method and a computer readable medium are described and claimed.

**Fig. 1**

**Description**

The present invention relates to a computer-implemented method of selecting a preferred training dataset from a plurality of training datasets for a target dataset. The invention further relates to a data processing system and a computer-readable medium.

Classification or labelling of data points of a target dataset is a core element of machine learning. The aim of the analysis is to identify or determine to which category of a set of categories a new observation or data point in a target dataset belongs. A category may also be referred to as a label or a class. For example, in sleep cycle detection, the classes may be sleep phases N1, N2 and N3, a rapid eye movement (REM) sleep phase and wakefulness (W). Sleep phases N1 to N3 may also be combined to a single non-REM or NREM sleep phase.

A target dataset may, for example, comprise a recording of physiological parameters of a person during a night including sets of physiological parameters for a plurality of points in time during the night. Each set of physiological parameters for one point in time of the target dataset would constitute an individual data point of the training dataset. The aim of the classification would be to identify the most likely class or label for each data point in the target dataset. For example, the data points in the target dataset could be classified as to whether they represent a non-REM or NREM sleep phase or a REM sleep phase.

A physiological parameter could, for example, be a heart rate of the person. The heart rate may be recorded using a single-lead electrocardiogram (ECG) or by monitoring the pulse rate through photoplethysmography (PPG) or ballistocardiography (BCG). From the recorded heart rate features may be extracted which are known to be useful for discriminating sleep phases in general or specifically REM sleep phases from NREM sleep phases. Examples of features that can be extracted are spectral components of the heart rate such as a low frequency component (0.04 Hz to 0.15 Hz) and a high frequency component (0.15 Hz to 0.5 Hz). The features extracted for one point in time of a dataset would represent a data point of a dataset.

The task of classifying the data points of the target dataset is performed by a machine learning algorithm which has been trained on a source or training dataset. Such a trained machine learning algorithm may also be referred to as a detector. A training dataset is a set of data points which have previously been classified into the respective categories or labeled, for example, by an expert. The source dataset is considered to represent ground truth.

An example of a machine learning algorithm that can be used for identifying a sleep phase based on one or more physiological parameters could, for example, be a support vector machine (SVM).

In the example of classifying the sleep phase based on the heart rate, a recorded dataset may be turned into a training dataset with labeled data points, for example, by recording a polysomnography (PSG) for a person while at the same time recording the heart rate using, for example, ECG, PPG or BCG. Based on the PSG a temporal representation of the sleep phases, also referred to as a hypnogram, is created, for example, by human experts or established algorithms. The hypnogram can be used to classify or label each data point in the source dataset to establish a ground truth.

A training dataset may undergo further preprocessing before it is used to train the actual machine learning algorithm.

The training dataset is then used to train the machine learning algorithm by inputting the training data to a parametrized model. The model output is compared with the ground truth labels of the training dataset and the parameters of the model/machine learning algorithm are modified in such a way that the model output agrees as much as possible with the ground truth.

Applying a machine learning algorithm for classifying data points in datasets may be complicated by the fact that the datapoints are distributed in a way that does not allow a linear separation of the data points. To overcome this issue, it is well known to transform a training dataset into a higher dimensional space where the datapoints can be linearly separated. For example, in a two-dimensional data set the data points of a first class may be distributed close to the origin, whereas the data points of a second class may be distributed on a circle around the origin. When these datapoints are transformed into a three-dimensional space, it may be possible to separate the classes by a plane. This would allow linear separation of the data points as a plane is a linear object.

However, since finding the correct transformation may be a complex if not impossible task, the use of kernels has been established. Kernels allow computing the inner product of data points in the multidimensional space without ever computing the actual coordinates of the data points in the higher dimensional space. Kernels thus allow extending a linear model to a non-linear problem. Finding a good estimate of a kernel is part of the training of the machine learning algorithm.

For a detector or trained machine learning algorithm to achieve good classification performances, the distribution of the source dataset, i.e., the dataset on which the detector was trained, must not differ from the distribution of the new unlabeled datapoints that shall be classified. In other words, for the detector to reliably categorize the data points of a target dataset it is essential that the data points in the training or source dataset and the target dataset follow a similar statistical distribution.

Common examples of differences between or shifts in distributions are prior shifts, covariate shifts and concept shifts. Prior shift refers to differences in the distribution of the labels. For example, in the case of a REM and NREM phase detection, a prior shift would refer to a shift in the distribution of REM and NREM labels, a covariate shift would refer to a change in the probability density distribution describing the data points in the respective datasets and a concept shift would refer to a difference in the relationship between an observation, i.e., the heart feature, and the corresponding label, i.e., the sleep phase.

When multiple versions of a machine learning algorithm are available, each trained on a different training dataset, it is known to use training set selection to determine which of the trained machine learning algorithms achieves the best classification performance on a newly recorded target dataset with an unknown distribution of data points that have not been labelled. In other words, training set selection would allow selecting a machine learning algorithm trained on a dataset with a similar distribution as the newly recorded dataset. Training set selection is an iterative process that relies on numerically solving an optimization problem. Hence, it is computationally inefficient and requires extensive computational and timing resource.

Hence, it can be considered an object of the present invention to provide a computationally efficient way of selecting a machine learning algorithm trained on a preferred training dataset from a plurality of training dataset that allows a high classification performance on a given newly recorded target dataset.

The problem underlying the present invention is solved by a computer-implemented method according to claim 1, a data processing system according to claim 12 and a computer-readable medium according to claim 13. Preferred embodiments are described in the dependent claims.

In a first aspect the problem is solved by a computer-implemented method of selecting at least one preferred training dataset from a plurality of training datasets for a target dataset. The target dataset T comprises a plurality of data points $X_T$ to be classified in a plurality of classes. Each training dataset S comprises a plurality of data points $X_S$ which have been classified into the plurality of classes. Each training dataset S has been used to train a reference detector and an output of the reference detector can be used for classifying data points of target datasets into the plurality of classes. Further, for each training dataset S a kernel or similarity function $k_S(X_{S,i}, X_{S,j})$ has been determined, wherein the kernel or similarity function provides a measure for the likelihood that two data points $X_{S,i}$ and $X_{S,j}$ of the training dataset S belong to a same class of the plurality of classes. The method comprises the steps of applying for each training dataset S the corresponding reference detector to the target dataset to obtain a reference output $Y_T^{\{S\}}$ for the data points $X_T$ of the target dataset, determining for each training dataset S an indicator of the performance of the reference detector trained on the training data set S

based at least on the reference output $Y_T^{\{S\}}$ the data points $X_T$ of the target dataset and the kernel or similarity function $k_S$ of the respective training dataset, and selecting at least one preferred training dataset from the plurality of training datasets based on the performance indicators determined for the plurality of training datasets.

Thus, the present invention provides a method for determining a preferred training or source dataset given a specific target dataset. To this end the method provides a means to determine how similar the distribution of the data points in the training dataset and the distribution of the data points in the target dataset are by relying on the unsupervised information in the data points of the target dataset and the kernel or similarity function.

As explained in more detail below, the unsupervised information of the kernel matrices determined based on the kernel or similarity function can be used to correct for data shifts between the target dataset and the respective training dataset. However, the same information can also be used to estimate whether a training dataset is a good fit for the target dataset. If the datasets are similarly distributed, not correcting for data shifts will be necessary. On other hands, if large data shifts occur between the target dataset and a respective training dataset, these changes will have to be corrected for. The larger the correction that is necessary, the less good will the distribution of a specific training dataset match the distribution of the target dataset. Further, many existing methods for selecting a preferred training dataset for a given target dataset rely on examining individual data points of the target dataset. The more data points of the target dataset are examined, the more time and computing resources are required. Hence, in practice only some datapoints of the target dataset will be analyzed. This reduces the quality of the training set selection.

In other words, in many settings a plurality of training datasets may be available. Each of these training datasets comprises a plurality of data points which have previously been classified into two or more classes. The classification of the data points has been performed based on, for example, expert knowledge and is considered ground truth. Also, each of the training dataset may have been preprocessed, for example, to center the data points of the training dataset or remove certain datapoints which cannot be classified into one of the two or more classes. Each of the plurality of training datasets has further been used to train a machine learning algorithm which can be used to classify the data points of other datasets into the respective classes. The trained machine learning algorithms are referred to as reference detectors.

Training the reference detectors may involve the use of testing datasets which also include data points that have previously been classified so that the classification represents ground truth. The parameters of the reference detectors are then tuned to obtain optimal classification of the data points in the test datasets.

As part of the training of the machine learning algorithm or reference detector, a kernel is determined which enables linear separation of the data points of the training dataset. For a given kernel the elements $K_{ij}$ of the Gram matrix $K$ are calculated for the data points $\boldsymbol{X}_S$ as $\boldsymbol{K_{ij}} = \boldsymbol{k}(x_{S,i}, x_{S,j})$ where $k_S(\cdot,\cdot)$ denotes the kernel function of the training dataset S. The Gram matrix $K$ can be considered a map of similarity: in the case of a unit-norm kernel, the closer $K_{ij}$ is to 1, the likelier it is for observations or data points $i$ and $j$ to belong to the same class.

For example, in the case of binary classification with a first class and a second class, the label $y_i$ of a data point $x_i$ would be $y_i = -1$, when the data point $x_i$ is in the first class, and $y_i = +1$, when the data point $x_i$ is in the second class. Further assuming an ideal classifier that directly returns the correct label or class for any given input, the elements $K_{ij}^* = y_i y_j$ of the ideal Gram matrix $K^*$ are +1 for data points coming from the same class and -1 for data points of different classes. Since during training the ground truth class or label of each data point is known, the ideal Gram matrix can be computed and the kernel function $k_S(\cdot,\cdot)$ can be tuned or optimized such that the Gram matrix $K$ closely resembles the ideal Gram matrix ideal Gram matrix $K^*$.

For engineering or tuning of the kernel, it is possible to determine the alignment A of a Gram matrix $K$ with the ideal Gram matrix $K^*$ as

$$A(K, K^*) = \frac{\langle K, K^* \rangle_F}{\sqrt{\langle K,K \rangle_F \langle K^*,K^* \rangle_F}},$$

where $\langle \cdot,\cdot \rangle_F$ denotes the Frobenius inner product and $K^* = YY'$. $Y$ denotes a vector of the ground truth labels $y_i$, i.e., the classes or labels of the data points of the respective training data set that have been determined previously. The more similar the ideal Gram matrix $K^*$ and the Gram matrix $K$ of the kernel are, the closer will the alignment be to 1. Hence, for tuning the kernel function, it is sufficient to maximize $A$ with regard to $K$.

In view of the above, the inventors have noted that the performance of a reference detector trained on a training dataset S, the kernel function $k_S(\cdot,\cdot)$ or another similarity function describing the similarity between the data points $\boldsymbol{X}_S$ of the training dataset S can be used to estimate labels $Y_T$ for the target dataset data points $\boldsymbol{X}_T$. These estimated labels $Y_T$ may also be referred to as the kernel output $Y_T$. A performance indicator can be determined from the so-determined kernel output and the output or labels predicted using the reference detector for the same training dataset. Exemplary ways of calculating the performance indicator are described as exemplary embodiments.

Since the measures of similarity or reference indicators are determined based on the kernel function and the target dataset datapoints $\boldsymbol{X}_T$, they advantageously provide a measure of similarity between the distribution of data points of the respective training dataset and the distribution of the data points of the target dataset.

The reference indicators determined for the plurality of training datasets are eventually used to select a preferred training dataset which is suitable for classifying the data points of the target dataset. For example, the training dataset with the highest reference indicator may be selected.

The method according to the present invention thus advantageously provides a numerical way of determining among a plurality of training datasets and corresponding reference detectors a training dataset and this reference detector which has a similar distribution of data points as the data points in the target dataset and, thus, accurately classifies the data points of the target datasets.

As compared to existing methods for training set selection, the method according to the present invention provides an analytical solution which does not rely on iteratively solving optimization problems. Hence, the method is computationally more efficient as it requires less computing power and less computing time as compared to existing method. Further, all data points of the target dataset are considered when determining the performance indicator of each training dataset without drastically increasing the required computational resources. Hence, in the present method there is no trade-off between a resource saving implementation and a high-precision implementation. In other words, the present invention is not used to select suitable points from one source of data points for a training dataset, but all data points are verified as to whether they combined provide a meaningful training dataset.

In a preferred embodiment a training dataset is only selected as a preferred training dataset if the performance indicator of the respective training dataset exceeds a predetermined threshold. Alternatively or additionally, no training dataset is selected if none of the performance indicators determined for a training dataset of the plurality of training datasets exceeds a predetermined threshold. Hence, the exemplary embodiment provides for a selection of the preferred training dataset based on thresholds which the performance indicator must exceed. For example, all training datasets exceeding a first threshold may be selected as preferred dataset for a specific target dataset or no training dataset is chosen if all performance indicators are below a threshold. In the latter case no preferred training dataset is found. The first and the second threshold may be the same threshold.

It is further preferred to determine the performance indicator for each training dataset S of the training datasets by calculating a correlation between the respective reference output $Y_T^{\{S\}}$ and a kernel output $Y_T$. The kernel output $Y_T$ is preferably calculated for each training dataset using $Y_T = \frac{K_{TS}Y_S}{\|K_{TS}Y_S\|}$, where $K_{TS}$ is a matrix with matrix elements $K_{i,j} = k_S(\boldsymbol{X}_{T,i}, \boldsymbol{X}_{S,j})$ and $Y_S$ is a classification of the data points of the respective training data, wherein $\boldsymbol{X}_{T,i}$ is a data point of the target dataset and $\boldsymbol{X}_{S,j}$ is a data point of the training dataset S.

For the present embodiment the inventors noted further that the previously discussed alignment measure can be extended to a kernel cross alignment

$$A_{KCA} = \frac{\langle K_{TS}, Y_T Y_S' \rangle_F}{\|K_{TS}\|_F \|Y_T Y_S'\|_F}$$ for assessing the similarity between the training dataset labels $Y_S$, target dataset labels $Y_T$ and the Gram matrix $K_{TS}$ representing the similarity of the data points of the training dataset and the target dataset. $F$ denotes the Frobenius norm of a matrix. The numerator of $A_{KCA}$ can be rewritten as

$$\langle K_{TS}, Y_T Y_S' \rangle_F = Y_T' K_{TS} Y_S.$$ For a given vector of training dataset labels $Y_S$ and a given Gram matrix $K_{TS}$, the kernel cross alignment $A_{KTS}$ can be maximized for $Y_T$ under the assumption that $\|Y_T\| = 1$. Under these assumptions, a solution for the target dataset labels can be obtained as

$$Y_T = \frac{K_{TS}Y_S}{\|K_{TS}Y_S\|}.$$ Hence, by using the unsupervised information contained in $K_{TS}$ (linking training dataset data points and target dataset data points), an improvement of the output of the supervised information given by $Y_S$ is obtained. This may also be referred to as Kernel-Cross Alignment Transfer Learning (KCATL) which forms by itself an inventive concept.

The inventors further noted that the estimated target data point labels $Y_T$ can be used to estimate how well the reference detector trained on the respective target dataset S will predict the labels of the target dataset. Since the labels obtained through KCATL correct for shifts in the distribution of the data points of the target dataset and the respective training dataset, the more the estimated target data point labels $Y_T$ will differ from the labels $Y_T^{\{S\}}$ determined using the reference detector. On the other hand, if the labels predicted using the transfer learning method are similar to the labels obtained using the reference detector, the distributions of the training dataset and the target dataset are similar. Hence, a correlation between these labels may serve as indicator of the performance of a training dataset and the reference detector trained on that training dataset for the target dataset.

Further, the method advantageously considers all data points of a target dataset and not only a selected few as in the prior art. Thus, there is no trade-off between accuracy and computational requirements.

Hence, when trying to select a preferred training dataset among a plurality of training datasets, the training dataset labels $Y_S$ and the kernel function $k_S$ are fixed. So in order to find an estimate of the target labels or kernel output $Y_T$, the Gram matrix $K_{TS}$ with the matrix elements $K_{i,j} = k_S(\boldsymbol{X}_{T,i}, \boldsymbol{X}_{S,j})$ can be used in the equation

$$Y_T = \frac{K_{TS}Y_S}{\|K_{TS}Y_S\|}$$ as an estimate. The correlation of the thereby obtained kernel output for a specific training dataset and the output obtained by using the reference detector on the target dataset can then advantageously be used as an indicator of the performance of the respective training dataset. The correlation can, for example, be calculated as a Pearson correlation coefficient.

Alternatively, the kernel output $Y_T$ is preferably calculated for each training dataset using $Y_T = \frac{K_{TT}Y_T^{\{S\}}}{\|K_{TT}Y_T^{\{S\}}\|}$, where $K_{TT}$ is a matrix with matrix elements $K_{i,j} = k_S(\boldsymbol{X}_{T,i}, \boldsymbol{X}_{T,j})$, wherein $\boldsymbol{X}_{T,i}$ and $\boldsymbol{X}_{T,j}$ are data points of the target dataset.

For the alternative embodiment, the inventors determined that an alignment of the kernel could also be achieved by determining $argmax \, Y_T'^{\{S\}} K_{TT} Y_T$ with respect to $Y_T$ under the assumption that $\|Y_T\| = 1$, where $K_{TT}$ is the Gram matrix computed between target observations which represent unsupervised information. This allows to calculate the labels of the target dataset as

$$Y_T = \frac{K_{TT}Y_T^{\{S\}}}{\|K_{TT}Y_T^{\{S\}}\|}.$$ Thus, by using unsupervised information contained in $K_{TT}$ and establishing a link between the target dataset $T$ and the training dataset $S$ through the output $Y_T^{\{S\}}$ of the reference detector, again an improvement of the output of the supervised information *is obtained.* This may also be referred to as Kernel-Target Alignment Transfer Learning (KTATL) which forms by itself an inventive concept.

The previously presented considerations also apply to this embodiment. If KTATL needs to accommodate for large shifts between the distributions of the target dataset and a training dataset, the labels calculated using KTATL will differ considerably from the labels obtained using the reference detector. On the other hand, if there are little to no shifts, the labels obtained using KTATL and the reference detector will be very similar. Hence, the correlation between the labels obtained using the transfer learning method and the reference detector may serve as an indication of the performance of the respective training dataset and the corresponding reference detector.

Further, the method advantageously considers all data points of a target dataset and not only a selected few as in the prior art. Thus, there is no trade-off between accuracy and computational requirements.

When selecting a preferred training dataset among a plurality of training datasets, the kernel function $k_S$ are fixed. So in order to find an estimate of the target labels or kernel output $Y_T$, the Gram matrix $K_{TT}$ with the matrix elements $K_{i,j} = k_s(\boldsymbol{X}_{T,i}, \boldsymbol{X}_{T,j})$ can be used in the

equation $Y_T = \dfrac{K_{TT}Y_T^{\{S\}}}{\left\| K_{TT}Y_T^{\{S\}} \right\|}$ as an estimate. The correlation of the thereby obtained kernel output for a specific training dataset and the output obtained by using the reference detector on the target dataset can then advantageously be used as an indicator of the performance of the respective training dataset.

In another preferred embodiment the performance indicator is determined for each training dataset as

an alignment $\dfrac{Y_T^{\{S\}}{}' K_{TT} Y_T^{\{S\}}}{\|K_{TT}\|_F \left\| Y_T^{\{S\}} Y_T^{\{S\}}{}' \right\|_F}$, wherein F denotes the Frobenius norm of a matrix and $K_{TT}$ is a matrix with matrix elements $K_{i,j} = k_s(\boldsymbol{X}_{T,i}, \boldsymbol{X}_{T,j})$, wherein $\boldsymbol{X}_{T,i}$ and $\boldsymbol{X}_{T,j}$ are data points of the target dataset.

In this preferred embodiment, the alignment is directly considered as performance indicator, by comparing the unsupervised information in $K_{TT}$ to the supervised information in the label-matrix $Y_T^{\{S\}} Y_T^{\{S\}}{}'$ -The alignment serves as a performance indicator which can advantageously be used to select a highly performing target dataset for each training dataset without having to solve an optimization problem.

Alternatively, the performance indicator may be determined for each training dataset as an alignment

$\dfrac{Y_T^{\{S\}}{}' K_{TS} Y_S}{\|K_{TS}\|_F \left\| Y_T^{\{S\}} Y_S{}' \right\|_F}$, wherein $F$ denotes the Frobenius norm of a matrix, $K_{TS}$ is a matrix with matrix elements $K_{i,j} = k_S(X_{T,i}, X_{S,j})$, where $\boldsymbol{X}_{T,i}$ is a data point of the target dataset $T$, $\boldsymbol{X}_{S,j}$ is a data point of the training dataset $S$, and $Y_S$ denotes the classification of the data points $\boldsymbol{X}_S$ of the training dataset $S$.. In a similar manner in the alternative embodiment, the alignment is directly considered as performance indicator, by comparing the unsupervised information in $K_{TS}$ to the supervised information in the label-matrix $Y_T^{\{S\}} Y_S{}'$ -The alignment serves as a performance indicator which can advantageously be used to select a highly performing target dataset for each training dataset.

In a preferred embodiment the reference detectors are support vector machines trained on the respective training datasets.

It is further preferred that the plurality of classes consists of two classes, wherein the reference output of the reference detectors for each of the training datasets is binarized and wherein the kernel output is binarized. The output may, for example, be binarized as +1 and -1.

In another preferred embodiment, the data points of the training datasets and the target dataset are physiological parameters and, in particular, features of a heart rate of a person and wherein the plurality of classes consists of two classes, the first class being a rapid eye movement, REM, sleep phase and the second phase being an non-REM sleep phase. Hence, in the preferred embodiment the method according to the present invention is preferably used to determine a preferred training dataset and the corresponding preferred reference for classifying a physiological signal. A particularly preferred physiological parameter may be features of the heart rate of a sleeping person which have been extracted from the heart rate in a preprocessing step. For example, the feature maybe different spectral components of the heart rate such as a low frequency component (0.04 Hz to 0.15 Hz) and a high frequency component (0.15 Hz to 0.5 Hz). Each feature for one point in time constitutes a data point of the dataset. The data points in the datasets can, for example, be classified into two classes. Preferably, they can be classified as representing either REM or non-REM sleep.

Preferably, only one preferred training dataset is selected based on the performance indicators and wherein the data points of the target dataset are classified based on the reference output determined by reference detector trained using the preferred training dataset. In the preferred embodiment the reference detector trained based on a single preferred training dataset is used to classify the data points of the target dataset.

Alternatively, two or more preferred training datasets are preferably selected based on the performance indicators, wherein an adapted detector is trained using a combination of the two or more preferred training datasets for classifying the data points of the target dataset into the plurality of classes and classifying the data points of the target dataset into the training dataset using the adapted detector.

Furthermore it is preferred to obtain for each training dataset the classification $Y_S$ of the data points of the training dataset by classifying the data points of the reference set with the reference detector trained on the training dataset. In other words, while the data points of the training datasets S have originally been classified into two or more classes by an external classifier such as an expert, for the purpose of selecting a preferred training dataset, the data points of each training dataset are reclassified using the reference detector obtained for the respective training dataset. This has been shown to improve the performance of the training dataset selection method described herein.

In a second aspect the problem underlying the present invention is solved by a data processing system comprising at least one data processing unit and a memory, wherein the at least one data processing unit is configured to perform a computer-implemented method according to any of the preceding embodiments.

The advantages of the data processing system correspond to the advantages of the embodiment of the computer-implemented method performed with the data processing system.

A computer readable medium including a plurality of instructions, which when executed by at least one processing unit, causes the at least one processing unit to execute a method according to any of the preceding embodiments.

The advantages of the computer readable medium correspond to the advantages of the embodiment of the computer-implemented method performed when the plurality of instructions is executed by the data processing system.

In the following an exemplary embodiment of a method for selecting a preferred training dataset will be described in more detail with regard to the drawings, wherein

Figure 1    shows a flow chart depicting the steps of an exemplary embodiment of a method for selecting a preferred training dataset among a plurality of training datasets,

Figure 2a   shows a diagram of a heart rate recorded for a sleeping patient over a period of time,

Figure 2b   shows a diagram of a recorded movement intensity of the patient of figure 2b over the same period of time,

Figure 3a   shows data points of a first training dataset in a three-dimensional space and a decision boundary of a first reference detector trained on the first training dataset,

Figure 3b   shows data points of a second training dataset in a three-dimensional space and a decision boundary of a second reference detector trained on the second training dataset,

Figure 4a   shows data points of a target dataset in a three-dimensional space and the decision boundary of the first reference detector applied to the target dataset,

Figure 4b   shows data points of the target dataset of Figure 4a in a three-dimensional space and the decision boundary of the second reference detector applied to the target dataset,

Figure 5    shows a diagram of the actual performance of a reference detector and a predicted performance determined using an exemplary embodiment of the present invention,

Figure 6    shows a diagram depicting the sleep phases of the target dataset of Figures 4a and 4b as ground truth, as determined by the first reference detector and as determined by the second reference detector and

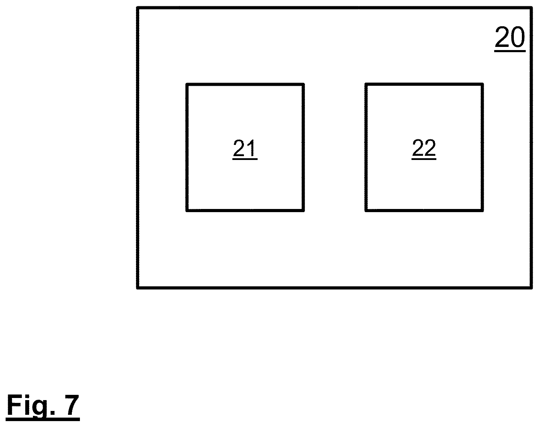

Figure 7    shows an exemplary embodiment of a data processing system.

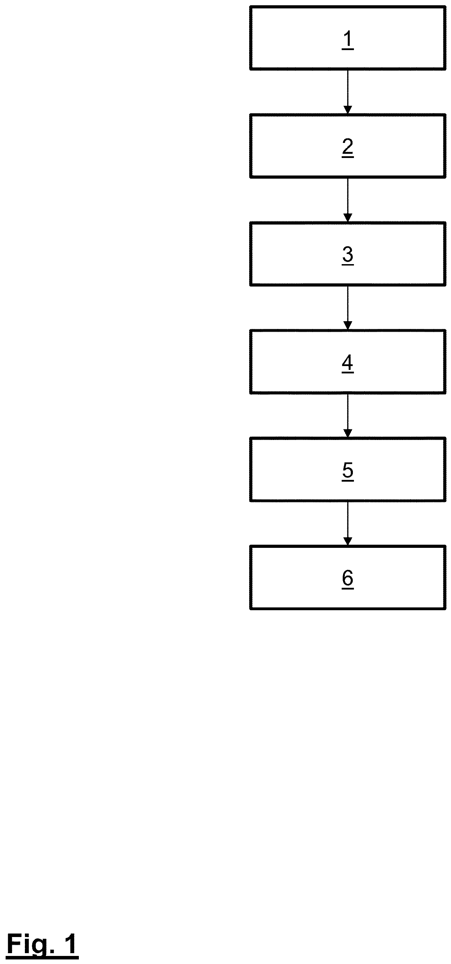

In the following an exemplary embodiment of a method for selecting a training dataset among a plurality of training datasets for classifying datapoints of a target dataset will be described in more detail with reference to the flow chart in Figure 1. In the exemplary embodiment the method is applied to the problem of classifying the sleep phases or stages of a person based on the heart rate of the person. In the exemplary embodiment, a preferred training dataset will be selected among a plurality of training datasets for determining as to whether data points in the target dataset correspond to a REM sleep phase or a non-REM sleep phase, i.e., any of sleep phases N1 to N3. However, the exemplary method according to the present invention can also be used to classify data points of other kinds of data, in particular, of other kinds of physiological data such as radiological images, bioanalyses, prescriptions, among others.

In a first step 1 of the method physiological parameters are recorded for a person from which a target dataset can be prepared. In the exemplary embodiment, the recorded physiological parameters are a heart rate of a patient and an intensity of the movements of the person. These physiological parameters have been recorded while the person was asleep. The heart rate may be recorded using a single-lead electrocardiogram (ECG) or by monitoring the pulse rate through photoplethysmography (PPG) or ballistocardiography (BCG).

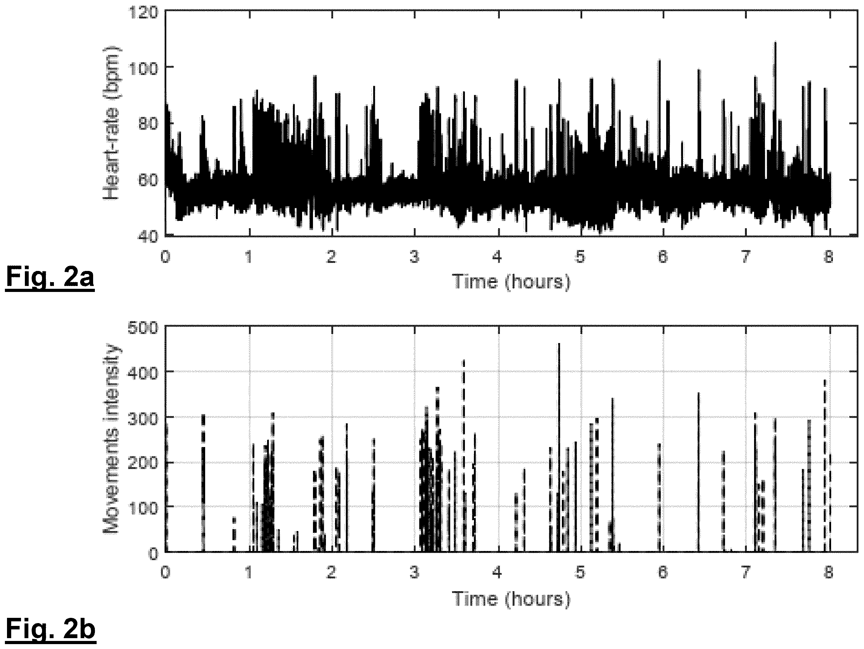

A visual representation of physiological parameters recorded for a training dataset is shown in Figures 2a and 2b. Figure 2a shows the heart rate in beats-per-minute (bpm) of the person recorded over a time of 8 hours. In Figure 2b the intensity of the movements or movements intensity of the person is shown in arbitrary units recorded. The intensity of the movements has been recorded over the same 8 hours as the heart rate.

In a second step 2 the target dataset is prepared from the recorded physiological parameters. To this end features are extracted from the recorded parameters that are known to be meaningful for the classification task. For example, for discriminating sleep stages in general and REM and non-REM sleep in particular spectral components of the heart rate such as a low frequency component (0.04 Hz to 0.15 Hz) and a high frequency component (0.15 Hz to 0.5 Hz) have proven to be useful features. The set of features extracted for one point in time together form a data point $X_{T,i}$ of the target dataset $T$. All data points of the target dataset are denoted with $X_T$. The data points of the target dataset may undergo further preprocessing which is not described here in more detail. For example, data points which pertain to different classes than the classes to be categorized may be removed (such as data points at which the person was awake) and the data points may be centered.

In a third step 3 the data points of the target dataset are classified using reference detectors trained on a plurality of training datasets from which a preferred training datasets and, therefore, also a preferred reference detector shall be selected using the exemplary embodiment of the method.

Each of the training datasets S comprises a plurality of data points $X_S$. The training datasets were obtained in a similar way as the datapoints of the target dataset, i.e., by recording physiological parameters of one or more persons while the person was asleep. Meaningful features were extracted from these physiological parameters. The set of features extracted for one point in time together form a data point $X_{S,i}$ of the training dataset S. The training datasets may undergo further preprocessing. All data points in the training datasets have further been classified or labeled by an existing classifier or by an expert. These labels are denoted with $Y_T$ and are considered ground truth.

Each training set has afterwards been used to train a machine learning algorithm, for example, a support vector machine, for classifying data points derived from a heart rate of a person as being recorded during a REM sleep phase or a non-REM sleep phase. A detailed description of the training of the machine learning algorithms is outside the scope of the present invention.

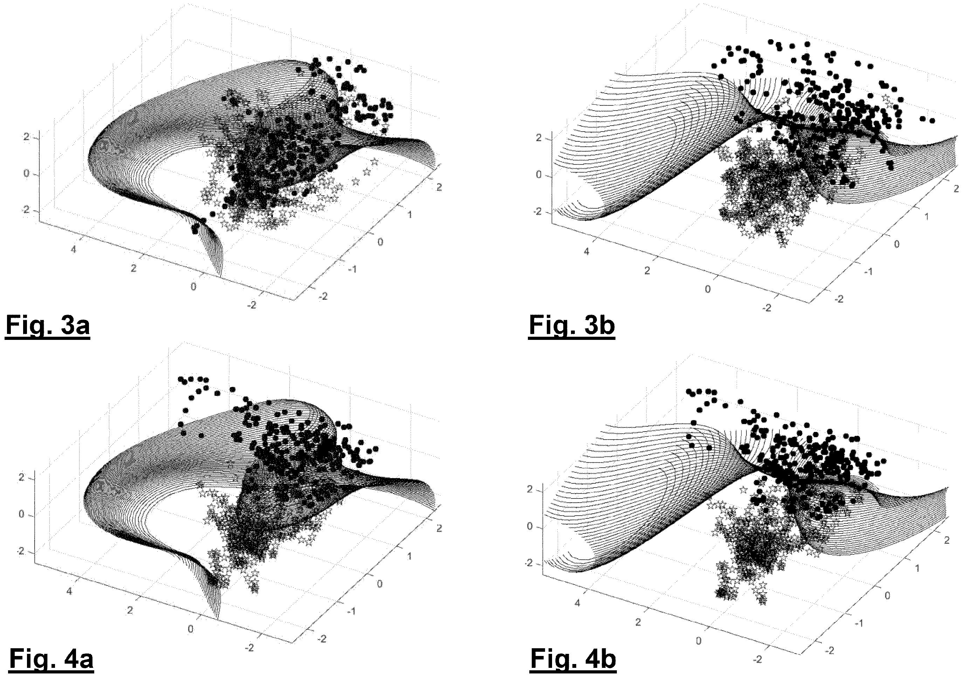

Figures 33a and 3b show the result of the training for two exemplary training datasets $S_1$ and $S_2$. In Figures 3a and 3b the data points of the two training datasets are depicted in a three-dimensional space spanned by the three features of the training datasets considered the most relevant for sleep phase detection. The dataset may comprise more than three features for each data point. Each data point is either denoted by a star or a dot depending on ground truth label of the respective data point. In other words, the symbol used to depict a data point indicates whether the data point has been previously classified as REM sleep or non-REM sleep.

The meshes in Figures 3a and 3b further show the decision boundary of the reference detector trained on the respective training dataset in continuous lines. All data points on one side of the decision boundary will be classified as REM sleep whereas all data point on the other point of the decision boundary will be classified as non-REM sleep. It is immediately apparent that the decision boundaries of the first and second training dataset differ considerably. Hence, depending on the distribution of the data points in the target dataset, the classification using either of two reference detectors the same data point may be classified differently.

This potential problem is illustrated further in Figures 4a and 4b. Here, the data points of the target dataset are shown in the three-dimensional feature space spanned by those three features of the target dataset which are considered to be the most useful for discriminating REM sleep from non-REM sleep. The feature space is the same used in Figures 3a and 3b. The data points illustrated in Figure 4a and 4b are also labelled according to their ground truth. Hence, all data points indicated by a star belong to the same class and all data points denoted by a dot belong to the same class.

Figures 4a and 4b further show meshes representing the decision boundaries of the reference detectors trained on training dataset $S_1$ (Figure 4a) and training dataset $S_2$ (Figure 4b), respectively. It can be clearly seen from the comparison of Figures 4a and 4b that due to the different distribution of data points of the training datasets $S_1$, $S_2$ and the target dataset $T$, the reference detectors perform differently on the target set.

Training of the machine learning algorithm also involves determining a kernel function $k_S(X_{S,i}, X_{S,j})$ where $X_{S,i}$ and $X_{S,j}$ are data points of the training set $S$. The kernel function is a measure for likelihood that the data points $X_{S,i}$ and $X_{S,j}$ are part of the same class. In other words, the kernel function indicates how likely it is, that data points are either both REM or non-REM data points or that one data point was recorded during a REM sleep phase while the other was recorded during a non-REM phase.

As previously indicated, for each training dataset $S$ the respective reference detector is used to classify the data points $X_T$ of the target dataset $T$. The output of the reference detector trained on training dataset $S$ is denoted by $Y_T^{\{S\}}$. A decision as to whether a data point was recorded during a REM sleep phase or a non-REM sleep phase is made based on a threshold. In case the output exceeds the threshold, a data point is classified as having been recorded during a REM sleep phase, otherwise as having been recorded during a non-REM sleep phase.

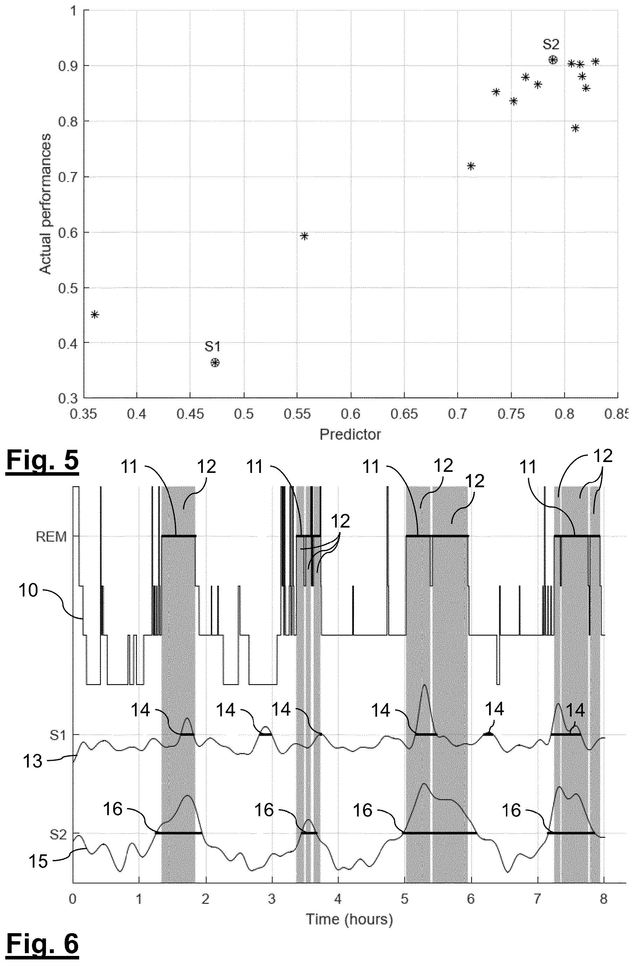

The results of the classification are depicted in Figure 5 where a top curve 10 shows the ground truth of the data points of the target dataset over time in hours. All datapoints forming bold horizontal bars 11 and being highlighted by a grey shade 12 are considered as having been recorded during a REM-sleep phase. All other datapoints are considered as having been recorded during a non-REM sleep phase.

The center curve 13 shows the output of the reference detector trained on training dataset $S_1$. Any part of the output exceeding the threshold indicated by the thick horizontal lines 14 is classified as REM sleep by the first reference detector, i.e., any data points that resulted in an output above the threshold are classified as REM sleep. All other data points are classified as non-REM sleep. Similarly, the bottom line 15 shows the output of the reference detector trained on training dataset $S_2$. Any part of the output exceeding the threshold indicated by the thick horizontal lines 16 is classified as REM sleep by the first reference detector, i.e., any data points that resulted in an output above the threshold are classified as REM sleep. All other data points are classified as non-REM sleep.

A comparison of the grey areas 12 with the thick black lines 14, 16 of the output 13, 15 of the reference detectors not only shows a discrepancy in the data points that have been classified as REM sleep and non-REM sleep. It is already apparent from the visual comparison that the reference detector trained on the training dataset $S_2$ appears to be more accurate than the reference detector trained on training dataset $S_1$.

Since the ground truth is commonly not available for target datasets, the present method includes a fourth step 4 in which for each training dataset S a kernel output is estimated based at least on the kernel function $k_S$ of the respective training dataset $S$ and the data points $X_T$ of the target dataset $T$. In the exemplary embodiment the kernel output was calculated as $Y_T = \dfrac{K_{TT} Y_T^{\{S\}}}{\left\| K_{TT} Y_T^{\{S\}} \right\|}$.

In a fifth step 5 a performance indicator is determined for each training dataset $S$ between the output $Y_T^{\{S\}}$ of the reference detector trained on training dataset $S$ when applied to the target dataset $T$. In the exemplary embodiment a correlation was calculated between the reference output $Y_T^{\{S\}}$ and the kernel output $Y_T$.

The measure of similarity is considered an indicator or predictor of the performance of the reference detector trained on a training dataset for the target dataset. In Figure 4 a chart is shown depicting for 15 reference detectors trained on different training datasets the predicted accuracy determined according to the exemplary embodiment on the abscissa (labelled "Predictor") and the actual performance of the reference detector on the ordinate. The predicted performance and actual performance of the reference detectors trained on the previously discussed first and second training datasets $S_1$ and $S_2$ are labelled with "Si" and "S2". As already expected from Figure 5, the reference detector trained on the second training dataset $S_2$ outperforms the reference detector $S_1$. This is accurately predicted by the performance predictor determined as part of the present method for selecting a training dataset without requiring the input of the ground truth.

Hence, the present invention advantageously allows predicting the accuracy of a reference detector trained on training dataset without requiring any knowledge of the ground truth of the target dataset. The prediction is made entirely using an analytical method and does not rely on numerically solving optimization problems. Compared to existing methods it is, therefore, computationally efficient. Also, it advantageously uses all data points of the target dataset and does not require a selection of data points to be analyzed. Hence, the accuracy of prediction does not depend on the selection of the target data points or the number of selected target data points.

In a final method step 6 a preferred training dataset $S$ is selected based on the performance indicator determined in the previous method step. In the exemplary embodiment the training dataset with highest performance indicator is chosen to select the reference detector for classifying the training dataset $T$. In case none of the performance indicators exceeds a threshold of, for example, 0.75, no training dataset is selected as none of them is considered a preferred training dataset.

The exemplary embodiment thus advantageously allows selecting a reference training dataset from a plurality of training dataset which will classify the datapoints of the target dataset with high accuracy. Advantageously, any element of the method can be calculated numerically. Further, since a rapid selection among a plurality of training datasets with low computational effort is possible, the method requires lower computational requirement than existing methods which rely on iterative approaches.

Finally, Figure 6 shows an exemplary embodiment of a data processing system 20 comprising a data processing unit 21 and a memory 22. The data processing unit 21 may comprise a plurality of distributed processing units and is configured to execute the exemplary embodiment of a method previously described.

**Claims**

1. A computer-implemented method of selecting a preferred training dataset from a plurality of training datasets for a target dataset,

   wherein the target dataset T comprises a plurality of data points $X_T$ to be classified in a plurality of classes,
   wherein each training dataset S comprises a plurality of data points $X_S$ which have been classified into the plurality of classes, wherein each training dataset S has been used to train a reference detector, wherein an output of the reference detector can be used for classifying data points of target datasets into the plurality of classes, and wherein for each training dataset S a kernel or similarity function $k_S(X_{S,i}, X_{S,j})$ has been determined, wherein the kernel or similarity function provides a measure for the likelihood that two data points $X_{S,i}$ and $X_{S,j}$ of the training dataset S belong to a same class of the plurality of classes,
   the method comprising the steps of

      applying for each training dataset S the corresponding reference detector to the target dataset to obtain a reference output $Y_T^{\{S\}}$ for the data points $X_T$ of the target dataset,
      determining for each training dataset $S$ an indicator of the performance of the reference detector trained on the training data set $S$ based at least on the reference output $Y_T^{\{S\}}$ the data points $\boldsymbol{X}_T$ of the target dataset and the kernel or similarity function $k_S$ of the respective training dataset, and
selecting a preferred training dataset from the plurality of training datasets based on the performance indicators determined for the plurality of training datasets.

2. The computer-implemented method according to claim 1, wherein a training dataset is only selected as a preferred training dataset if the performance indicator of the respective training dataset exceeds a predetermined threshold and/or
wherein no training dataset is selected if none of the performance indicators determined for the training datasets of the plurality of training datasets exceeds a predetermined threshold.

3. The computer-implemented method according to any of the preceding claims, wherein the performance indicator is determined for each training dataset $S$ of the training datasets by calculating a correlation between the respective reference output $Y_T^{\{S\}}$ and a kernel output $Y_T$,
wherein the kernel output $Y_T$ is calculated for each training dataset using $Y_T = \dfrac{K_{TT}Y_T^{\{S\}}}{\left\|K_{TT}Y_T^{\{S\}}\right\|}$, where $K_{TT}$ is a matrix with matrix elements $K_{i,j} = k_S(X_{T,i}, X_{T,j})$, wherein $\boldsymbol{X}_{T,i}$ and $\boldsymbol{X}_{T,j}$ are data points of the target dataset or
wherein the kernel output $Y_T$ is calculated for each training dataset using $Y_T = \dfrac{K_{TS}Y_S}{\|K_{TS}Y_S\|}$, where $K_{TS}$ is a matrix with matrix elements $K_{i,j} = k_S(\boldsymbol{X}_{T,i}, \boldsymbol{X}_{S,j})$ and $Y_S$ is a classification of the data points of the respective training data, wherein $\boldsymbol{X}_{T,i}$ is a data point of the target dataset and $\boldsymbol{X}_{S,j}$ is a data point of the training dataset $S$.

4. The computer-implemented method according to claim 1 or 2, wherein the performance indicator is determined for each training dataset $S$ as an alignment $\dfrac{Y_T^{\{S\}t}K_{TT}Y_T^{\{S\}}}{\|K_{TT}\|_F\left\|Y_T^{\{S\}}Y_T^{\{S\}t}\right\|_F}$, wherein $F$ denotes the Frobenius norm of a matrix, t the transpose operator and $K_{TT}$ is a matrix with matrix elements $K_{i,j} =$

$k_S(\boldsymbol{X}_{T,i}, X_{T,j})$, wherein $\boldsymbol{X}_{T,i}$ and $\boldsymbol{X}_{T,j}$ are data points of the target dataset.

5. The computer-implemented method according to claim 1 or 2, wherein the performance indicator is determined for each training dataset as an alignment $\dfrac{Y_T^{\{S\}t}K_{TS}Y_S}{\|K_{TS}\|_F\left\|Y_T^{\{S\}}Y_S{}^t\right\|_F}$, wherein $F$ denotes the Frobenius norm of a matrix, $K_{TS}$ is a matrix with matrix elements $K_{i,j} = k_S(\boldsymbol{X}_{T,i}, \boldsymbol{X}_{S,j})$, wheree $\boldsymbol{X}_{T,i}$ is a data point of the target dataset $T$, $\boldsymbol{X}_{S,j}$ is a data point of the training dataset $S$, and $Y_S$ denotes the classification of the data points $\boldsymbol{X}_S$ of the training dataset $S$.

6. The computer-implemented method according to any of the preceding claims, wherein the reference detectors are support vector machines trained on the respective training datasets.

7. The computer-implemented method according to any of the preceding claims, wherein the plurality of classes consists of two classes, wherein the reference output of the reference detectors for each of the training datasets is binarized and wherein the kernel output is binarized.

8. The computer-implemented method according to any of the preceding claims, wherein the data points of the training datasets and the target dataset are physiological parameters and, in particular, features of a heart rate of a person and wherein the plurality of classes consists of two classes, the first class being a rapid eye movement, REM, sleep phase and the second phase being an non-REM sleep phase.

9. The computer-implemented method according to any of the preceding claims, wherein only one preferred training dataset is selected based on the performance indicators and wherein the data points of the target dataset are classified based on the reference output determined by reference detector trained using the preferred training dataset.

10. The computer-implemented method according to any of claims 1 to 8, wherein two or more preferred training datasets are selected based on the performance indicators, wherein an adapted detector is trained using a combination of the two or more preferred training datasets for classifying the data points of the target dataset into the plurality of classes and classifying the data points of the target dataset into the training dataset using the adapted detector.

11. The computer-implemented method according to any of the preceding claims, wherein for each training dataset the classification $Y_S$ of the data points of the training dataset is obtained by classifying the data points of the reference set with the reference detector trained on the training dataset.

12. A data processing system (20) comprising at least one data processing unit (21) and a memory (22), wherein the at least one data processing unit (21) is configured to perform a method according to any of the preceding claims.

13. A computer readable medium including a plurality of instructions, which when executed by at least one processing unit (21), cause the at least one processing unit (21) to execute a method according to any of claims 1 to 11.

**Fig. 1**

**Fig. 2a**

**Fig. 2b**

Fig. 3b

Fig. 4b

Fig. 3a

Fig. 4a

14

**Fig. 5**

**Fig. 6**

**Fig. 7**

**EP 4 060 676 A1**

<table>
<tr><td colspan="4">**EUROPEAN SEARCH REPORT**</td><td>**Application Number**<br>EP 21 16 2964</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/110755 A1 (CAPODILUPO JOHN VINCENZO [US] ET AL) 18 April 2019 (2019-04-18) * abstract * ----- | 1-13 | INV.<br>G16H50/00 |
| | | | **TECHNICAL FIELDS SEARCHED (IPC)**<br>G16H |

The present search report has been drawn up for all claims

| Place of search | Date of completion of the search | Examiner |
|---|---|---|
| Munich | 2 August 2021 | Krawaritis, Achilles |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 060 676 A1**

## ANNEX TO THE EUROPEAN SEARCH REPORT
## ON EUROPEAN PATENT APPLICATION NO.

EP 21 16 2964

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2021

| Patent document cited in search report | Publication date | Patent family member(s) | Publication date |
|---|---|---|---|
| US 2019110755 A1 | 18-04-2019 | EP 3687392 A2<br>US 2019110755 A1<br>WO 2019079503 A2 | 05-08-2020<br>18-04-2019<br>25-04-2019 |

EPO FORM P0459

For more details about this annex : see Official Journal of the European Patent Office, No. 12/82